(19)

**Europäisches Patentamt**

**European Patent Office**

**Office européen des brevets**

(11) **EP 4 059 430 A1**

(12) **EUROPEAN PATENT APPLICATION**

(43) Date of publication:
**21.09.2022 Bulletin 2022/38**

(21) Application number: **21162472.1**

(22) Date of filing: **15.03.2021**

(51) International Patent Classification (IPC):
**A61B 5/305** (2021.01)      **A61B 5/318** (2021.01)
**A61B 5/00** (2006.01)

(52) Cooperative Patent Classification (CPC):
**A61B 5/305; A61B 5/318; A61B 5/725**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Koninklijke Philips N.V.**
**5656 AG Eindhoven (NL)**

(72) Inventor: **FRANCK, Christoph**
**5656 AE Eindhoven (NL)**

(74) Representative: **Philips Intellectual Property & Standards**
**High Tech Campus 52**
**5656 AG Eindhoven (NL)**

(54) **PHYSIOLOGICAL MEASUREMENT DEVICE COMPRISING DIGITAL FILTER AND METHOD**

(57)    The present invention relates to a physiological measurement device (11) comprising a plurality of input channels (23), at least one of the input channels including a digital section configured to process at least one digital signal ($d_1$, $d_2$) representing an analog signal ($e_1$, $e_2$) present at an electrode (13) assigned to the respective channel, a signal combiner (31) configured to calculate at least one vector signal (x) from at least two processed digital signals ($df_1$, $df_2$). To achieve improved common-mode interference mitigation, it is proposed that the digital section of at least one input channel (23) comprise a digital filter (29) configured to filter the digital signal ($d_1$, $d_2$) based on a set of filter coefficients ($G_1$, $G_2$) and a processor (33) configured for calculating the set of filter coefficients ($G_1$, $G_2$) based on the vector signal ($x_1$).

FIG.1

## Description

FIELD OF THE INVENTION

The present invention relates to a physiological measurement device and a respective method. Such device may comprise a plurality of input channels, at least one of the input channels including a digital section configured to process at least one digital signal representing an analog signal present at an electrode assigned to the respective channel and a signal combiner configured to calculate at least one vector signal from at least two processed digital signals.

BACKGROUND OF THE INVENTION

The combiner of the above-named device combines the at least processed digital signal to the vector signal so that common mode interferences present in the processed digital signals are mitigated.

A known issue when performing electrophysiological measurements such as electrocardiography (ECG), Electroencephalography (EEG) Electromyography (EMG) is that a signal path of each electrode has a slightly different transfer function due to component tolerances, different external cables, and the electrical properties of the skin-electrode interface, etc. As a consequence, the common-mode signal can be cancelled only partially.

The published patent application DE 10 2014 214 994 A1 describes a differential voltage measurement system that comprises an analog variable impedance element such as an RC-circuit that can be controlled for the purpose of impedance matching of two inputs of the measurement system. Moreover, combination of this impedance matching approach with a Right Leg Drive (RLD) technique is proposed to further reduce common mode interference.

SUMMARY OF THE INVENTION

It is an object of the present invention to provide a physiological measurement device, a physiological measurement method and a respective computer program product that allow for improved common-mode interference mitigation. Moreover, a cost-efficient implementation of a signal acquisition front-end should be possible without compromising the effectiveness of common-mode interference mitigation.

In a first aspect of the present invention a physiological measurement device is presented comprising a plurality of input channels, at least one of the input channels including a digital section configured to process at least one digital signal representing an analog signal present at an electrode assigned to the respective channel, a signal combiner configured to calculate at least one vector signal from at least two processed digital signals, wherein the digital section of at least one input channel, preferably each input channel of the plurality of input channels, comprises a digital filter configured to filter the digital signal based on a set of filter coefficients and a processor configured for calculating the set of filter coefficients based on the vector signal.

In further aspects of the present invention, there are provided a corresponding method, a computer program which comprises program code means for causing a computer to perform the steps of the method disclosed herein when said computer program is carried out on a computer as well as a non-transitory computer-readable recording medium that stores therein a computer program product, which, when executed by a processor, causes the method disclosed herein to be performed.

Preferred embodiments of the invention are defined in the dependent claims. It shall be understood that the claimed device, method, computer program and medium have similar and/or identical preferred embodiments as the claimed system, in particular as defined in the dependent claims and as disclosed herein.

The present invention is based on the idea that characteristics of the vector signal can be considered for adapting the digital filter of a channel of the measurement device rather than focusing on channel-specific characteristics like impedance. By adapting the set of coefficients of at least one digital filter of one channel based on the characteristics of the vector signal, the common-mode interference can be particularly well mitigated. As a consequence, medical personnel operating the measurement device will see cleaner measured curves (e.g. EEG or ECG curves) having less disturbing noise.

The use of digital filtering allows for compensating time skew between digital signals of different channels. Such time skew may be introduced by cost-efficient Analog to Digital Converter (ADC) architectures that use a single ADC to subsequently digitize multiple analog signals related to different channels. In other words, the claimed invention allows to implement a cost-efficient signal acquisition frontend without compromising common-mode mitigation effectiveness. The claimed device, method and program operate so effectively that the RLD technique or similar methods are not needed in order to archive good measurement results.

In an exemplary implementation, calculating the set of filter coefficients may be based on the unfiltered digital signal or samples thereof.

Moreover, calculating the set of filter coefficients can be based on a current set of filter coefficients. When calculating one or more new sets of filter coefficients for one or more digital filters based on the unfiltered digital signal and the current set of filter coefficients. An improvement step can be performed to adjust the currently used filter coefficients. This improvement step may be carried out during normal operation of the measurement device, e.g. during acquisition of a patient's ECG, EEG or EMG curves. Separate calibration or usage of special measurement signal is not needed.

Preferably, calculating the set of filter coefficients is performed repeatedly for each newly available sample of the digital signal or for a block of multiple subsequent samples of the digital signal. During operation of the measurement device, the common-mode rejection behavior will thus improve gradually while the filter coefficients are successively adjusted.

The improvement step may, e.g., include an optimization run. In other words, calculating the set of filter coefficients may comprise performing an optimization step, the measure constituting a target function and the set of filter coefficients constituting input variables of the target function. Although the herein described examples mainly use a cost function to be minimized - the target function may thus be a cost function - other types of target function may be applied, e.g. a utility function that needs to be maximized.

The inventor has realized that measures, in particular statistical measures, can described features of the vector signal that allow for distinguishing between a desired physiological measurement signal and common-mode interference. To allow for improved distinguishing the desired signal and the common-mode interference from each other, it is possible that the target function comprises a weighted sum of multiple measures.

To exclude undesired digital filter configurations form the optimization solution space, in a certain implementation, performing the optimization step may be subject to at least one constraint related to a certain set of filter coefficients. Such constraint may includes at least one equality constraint and/or at least one inequality constraint.

To avoid solutions where, e.g., the common-mode inference is filtered by the digital filters rather than eliminated by the signal combiner, the optimization step may be subject to at least one constraint related to a similarity measure characterizing a similarity of two different sets of filter coefficients.

Alternatively or in addition to the constraint related to the similarity measure, the target function may comprise the similarity measure characterizing a similarity of two different sets of filter coefficients.

Preferably, the processor may be configured to calculate multiple vector signals from the filtered digital signals, wherein the digital section of one channel includes at most one digital filter. The individual digital filters are thus specific to one channel but not necessarily specific to a certain vector signal.

In an exemplary implementation, the processor is configured to receive an indication from a back end and to determine the at least one measure and/or the target function based on the received indication. The back end may be part of the measurement device or included into a separate device. The indication allows to adapt the measurement device to special measurement situation that may be automatically detected by the back end and/or determined by the back end based on human user input. This adaptation may include selecting a target function and/or or composing a target function based on a set multiple measure. In particular, the weights of the weights sum of the multiple measures may be determined based on the indication.

For example, the signal combiner may be configured to calculate the vector signal from a further vector signal ($x_3$). In an ECG application, the further vector signal may correspond to a Wilson Central Terminal (WCT). The at least one vector signal calculated from the further vector signal may correspond to a lead related to a chest electrode.

The measurement device may include a signal acquisition frontend that includes the individual input channel comprising analog input circuitry including analog filters, ADC, the digital filters, the signal combiner and the processor. Optionally, the back end may also be included into the measurement device. The measurement device may be an ECG, an Electroencephalography (EEG), or an Electromyography (EMG) device for medical (diagnosis, patient monitoring, etc.) or non-medical (fitness trackers, smart watches, etc.) applications.

BRIEF DESCRIPTION OF THE DRAWINGS

These and other aspects of the invention will be apparent from and elucidated with reference to the embodiment(s) described hereinafter. In the following drawings

Fig. 1 shows a block diagram of a physiological measurement device according to a first example;
Fig. 2 shows a flowchart of a method to operate the device of Fig. 1;
Fig. 3 shows an ECG measurement device according to a second example and electrodes connected to this device;
Fig. 4 shows a block diagram of an ECG signal acquisition content of the measurement device shown in Fig. 3;
Fig. 5 shows a block diagram of a ECG signal acquisition signal front end according to a third example; and
Fig. 6 shows a diagram of signals within a measurement device over time.

DETAILED DESCRIPTION OF EMBODIMENTS

While the invention has been illustrated and described in detail in the drawings and foregoing description, such illustration and description are to be considered illustrative or exemplary and not restrictive; the invention is not limited to the disclosed embodiments. Other variations to the disclosed embodiments can be understood and effected by those skilled in the art in practicing the claimed invention, from a study of the drawings, the disclosure, and the appended claims.

In the claims, the word "comprising" does not exclude other elements or steps, and the indefinite article "a" or "an" does not exclude a plurality. A single element or other unit may fulfill the functions of several items recited in the claims. The mere fact that certain measures are recited in mutually different dependent claims does not indicate that a combination of these measures cannot be used to advantage.

A computer program may be stored/distributed on a suitable non-transitory medium, such as an optical storage medium or a solid-state medium supplied together with or as part of other hardware, but may also be distributed in other forms, such as via the Internet or other wired or wireless telecommunication systems.

Any reference signs in the claims should not be construed as limiting the scope.

Electrophysiological measurements like electrocardiography, electroencephalography and electromyography work by measuring voltages that appear between certain points on a patient due to physiological cellular activity. The signals of interest (wanted signals or desired signals) are usually small - in the millivolt to microvolt range - and typically only the difference in electrical potential between the electrode positions is relevant. However, due to factors like electromagnetic interference (e.g. from power lines or electrical devices in close proximity of or in contact with the patient), the electrical potential of the patient changes over time, which leads each electrode picking up a mixture of the differential-mode physiological signal of interest and a non-physiological, undesired common-mode signal.

The difference between two electrode locations, or more general, any linear combination of electrode potentials where the sum of weighting coefficients is zero, is usually referred to as a vector signal or just a "vector". In the ideal case, the common-mode signal disappears completely when the potential difference between two electrodes is formed by calculating the vector signal.

However, in a real implementations, each electrode has a slightly different transfer function, which is defined by the quality of the patient/electrode interface, the properties of the cable between the electrode and the measurement device, component tolerances in the input circuitry of the measurement device, and the properties of the analog-to-digital conversion like time skew (in case of sequential sampling) and slightly different transfer functions if multiple analog-to-digital converters (ADCs) are used. The differences in the transfer functions can lead to a significant part of the undesired common-mode signal appearing in the vector as what is called common-mode interference.

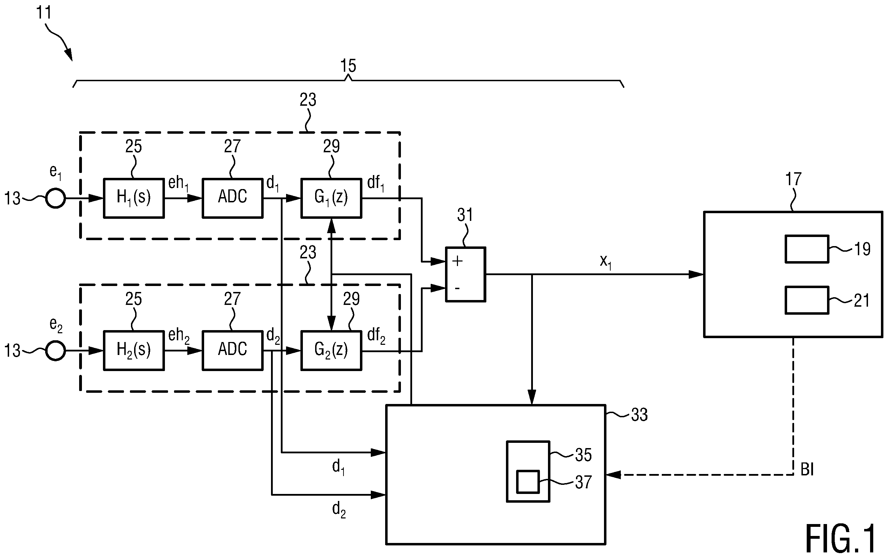

Fig. 1 shows a block diagram of a simple electrophysiological measurement device 11 having two electrodes 13. The measurement device 11 may comprise a signal acquisition front end 15 and an analysis back end 17. The front end 15 is configured to process analog signals $E_1$, $E_2$ inputted by the front end 15 via the electrodes 13 and to generate a vector signal x therefrom.

The back end 17 is operable for receiving the vector signal x from the front end 15 and therefrom further operations such as further signal analyses and/or signal classification, in particular rhythm classification in case of a ECG device. Moreover, the back end 17 may include user interface elements such as a screen and/or a keyboard, or the like to display the vector signal x in original or post process form or to display signal analysis or classification results to a user of the device 11. The analysis back end 17 may comprise a back end processor 21, which may include a computer program to perform the above-mentioned operations of the back end 17. Other than the shown example, a measurement device may be provided that does not include the back end 17. For example, the back end may be remote with respect to the measurement device.

The front end 15 comprises input channels 23 dedicated to the individual electrodes 13. The exemplary device shown in Fig. 1 has two input channels 23 that can be connected to two electrodes 13. The disclosure of the present patent application is not limited to this comparatively simple example. It can be used in connection with measurement devices having a larger number of input channels 23 and thus supporting a higher number of connected electrodes 13.

Each input channel 23, includes an analog section comprising circuitry for inputting the analog signal $e_1$, $e_2$ from a respective electrode 30 and to perform basic signal preconditioning of the respective analog signal. To this end, the analog section may include an analog filter 25 having a transfer function $H_i(s)$ where i denotes a channel number. The analog filter 25 may be designed as an antialiasing filter to prevent aliasing effects when digitizing the analog signal and/or may be designed to adapt a voltage or energy range of the respective signal $e_i$ to an input range of an analog-to-digital converter (ADC 27) connected to the analog filter 25. The ADC 27 is operable for converting an analog signal $e_{hi}$ outputted by the analog filter 25 of the analog section into a corresponding digital signal $d_i$, thereby forming an interface between the analog section of the respective channel 23 and a digital section of this channel 23. The digital section of the channel 23 includes a digital filter 29 adapted to filter the digital signal $d_i$ to obtain a filtered digital signal $d_{fi}$. The digital filter 29 may include a Finite Impulse Response (FIR) filter defined by a set of filter coefficients $G_i$.

In the example shown in Fig. 1, one ADC 27 is provided for each channel 23. However, it is also possible to implement less ADCs 27 in the front end 15 than channels 23. In other words, at least one ADC may be shared among multiple channels 23. In a practical implementation, one ADC may include an analog input multiplexer, inputs of which being connected to outputs of the analog filters 25 of different channels 23 to sequentially forward the individual filtered analog signals $e_{hi}$ to the ADC so that these signals $e_{hi}$ are sequentially sampled and digitized by the shared ADC. Sequential sampling and digitizing typically introduces a time skew between the digital signals $d_i$ of different channels 23 since the respective analog signals $e_i$ are sampled successively at different time instances.

The front end 15 further includes a combiner 31 configured to combine at least two filtered digital signals $df_i$, thereby obtaining a vector signal $x_1$. In the shown example, the combiner 31 is adapted to subtract the second filtered digital signal $df_2$ from the first digital signal $df_1$. Accordingly, weights used to calculate the vector signal $x_1$ are 1, and -1, respectively.

The signal processing front end 15 further comprises a front end processor 33. The processor 33 may include a programmable digital computer 35 including a memory device 37. A computer program may be stored on the memory device 37 when is programmed to cause the computer 35 to execute the herein described processing operations. Such computer program may also be stored on any storage device (flash storage, magnetic storage, etc.) that is not part of the processor 33. A program may be even made available for download over a communication network such as the internet. It should also be noted that in some implementations signal processing operations performed by blocks of Fig. 1 showed outside of the processor 33, in particular operations performed by the digital section of the input channels 23, may also be performed by the computer 35 of the processor 33.

The front end processor 33 is configured, e.g. programmed, to receive the vector signal $x_1$ and to calculate the sets $G_1$, $G_2$ of filter coefficients for the digital filters 29 based on the vector signal. Furthermore, the processor 33 may calculate the sets of filter coefficients also based on samples and $d_1$, $d_2$ of the unfiltered digital signal output by the ADC 27 of individual channels 23. Moreover, the processor 33 may be configured to calculate the sets of coefficients of filters 29 iteratively; that is the calculation of the sets of filter coefficients will further be based on current sets of filter coefficients.

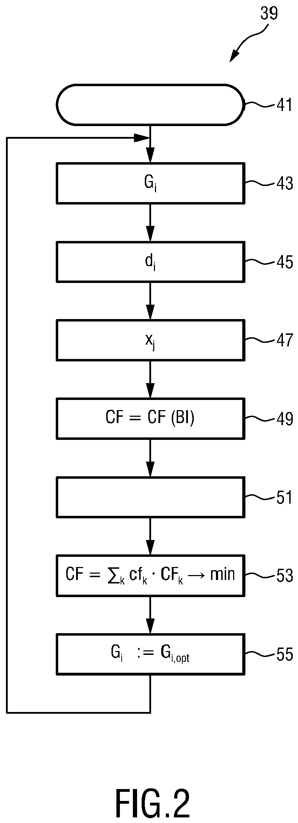

Fig. 2 shows a method 39 that can be carried out by the front end processor 33. In particular, the program stored in the memory device 37 may be programmed to execute the method 39.

After a start 41 of the method, a step 33 is executed for reading current sets of filter coefficients $G_1$, ..., $G_N$, where N denotes the number of channels 23. In the example shown in Fig. 1, the number of channels is two thus N = 2.

Step 45, which may follow step 43, reads a sequence of samples of the unfiltered digital signal where $d_i$ is a sequence of samples of digital signal i, i = 1, ..., N. The unfiltered digital signal $d_i$ is the digital signal present in the respective channel 23 that has not yet been processed by the digital filter 29. For example, the unfiltered digital signal may correspond to the signal generated by the respective ADC 27. Step 45 may capture exactly one sample per channel 23 (on-line mode). Alternatively, step 45 may collect a sequence of multiple samples for each channel 23 (batch mode). For the sake of simplicity, the single sample of a certain channel 23 or the sequence of multiple samples of a certain channel e will be referred to as $d_i$. Accordingly, in step 45 the values $d_1$, ..., $d_N$ are obtained.

In a step 47 of the method 39, subsequent samples $x_j$ of one or more output vectors are $x_j$ collected. The example of Fig. 1 is only one vector signal $x_1$. However, in the general case, the method 39 may consider M vector signals $x_1$, ..., $x_M$. The number of samples collected for the individual vector signals $x_j$ is preferably so selected that statistical measures can be calculated from the individual sequences $x_j$ in a reasonable way.

Step 49 of the method 33 selects a cost function CF based on an indicator BI received from the back end processor 17. The indicator BI may include information determined by the back end processor 17 derived from long-term, high level signal analysis such as rhythm classification or results of automatic analysis or classification performed by the back end processor 17. The indicator BI thus includes information usable by the processor 33 to appropriately select and/or compose the cost function CF.

As will be described in detail below, the method 39 uses statistical properties of at least one vector signal $x_j$ to determine if and to what the extent the vector signal is adversely affected by disturbances such as common mode interference. Because the measurement device is used to process a wide range of normal and pathological waveforms, such as ECG waveforms, adapting the cost function CF to certain pathological patterns like tachycardia or ventricular fibrillation can improve the performance of the method 39.

In an ECG recorder, for instance, the component responsible for signal acquisition and conditioning (front end 15) may be not aware of this long-term signal analysis. Thanks to the indicator BI received from the front end processor, the cost function CF can be adapted to best distinguish signals corresponding to the currently identified cardiac rhythm from common-mode interference sources. As an alternative to the dynamic cost function selection, adaptation or composition of step 49, a fixed cost function CF may be used and step 49 may be omitted.

The cost function CF is used to measure the statistical similarity of a vector signal $x_j$ with a desired signal such as an ECG signal, or its dissimilarity with expected common mode interference signals of other disturbances. Components of the cost function may include statistical measures of the (numerically calculated) derivative of the vector signal. The derivative considers the time structure, whereas measures like kurtosis and skewness consider the histogram and are unchanged when the order of the samples is changed. As an example, a measure that quantifies the similarity of the distribution of the vector signal to that of a sine wave would show a high amount of similarity when the input signal is a random permutation of the samples of a sine wave, but the difference would become clear if the derivative of the vector signal is considered.

In an implementation, the values of the vector signal used for calculating the statistical measures may be normalized first, i.e. shifted so their mean is zero, and scaled so their standard deviation is one. Depending on the number of available samples, the normalization may be approximate. Normalization makes the calculated measures (and their derivatives) scale-invariant.

In the following, possible statistical measures to be calculated based on the collected subsequent samples of the vector signal determined in step 47 will be described.

To measure the density of a vector signal $x_j$ the L1-norm of $x_j$ may be used. For example, a vector signal $x_j$ used in an ECG application has usually a sparse density where many samples are at least close to zero except for some pathological patterns. A common mode signal, however is dense because it usually has a sinusoidal periodic or quasi-periodic pattern.

To measure the total variation of the vector signal $x_j$ over time the L1-norm of the differentiated vector signal $x_j$ may be used. The differentiated vector signal can be calculated by subtracting subsequent values from each other (by subtracting a respective preceding value from each value). The total variation of e.g. an ECG vector signal is low, whereas a total variation of a common mode interfering signal is high.

To measure an amplitude, the L2-norm (Euclidian norm) or the Loo-norm may be used. Desired ECG signals have a limited amplitude that is typical in the range below 10 mV p-p whereas the amplitude of a disturbing common mode signal is often larger than 10 mV p-p.

Moreover, measures related to a probability density distribution of the vector signal $x_j$ can be used. When using the Kurtosis of the probability density distribution as a cost function, a vector signal usually having significant supergaussian/leptokurtic properties can be distinguished from disturbing common mode signals usually having subgaussian/platykurtic properties.

As an alternative to the Kurtosis or in addition to the Kurtosis, the negentropy measure (or an approximation thereof) calculated for the probability density distribution can be used as a cost function.

To measure the symmetry of the probability density distribution, the skewness measure of the probability density function may be used as a cost function. Instead of the skewness, an asymmetry heuristics that is more robust to outliers may be used such as $x_j * \exp(-x_j^2/2)$.

One of the above-mentioned cost functions can be used as the cost functions CF do calculate optimized sets $G_i$ of filter coefficients. Alternatively, the cost function CF may be composed by a weighted addition of multiple cost functions, $CF = \sum_{k=1}^{K} cf_k \cdot CF_k$, where $CF_k$ is one cost function, $cf_k$ the respective weight and K the number of cost functions $CF_k$ used to compose the final cost function CF. For example, the overall cost function CF could be chosen to be the L1-norm minus skewness of a resulting vector $x_j$. A negative gradient (considering the optimized sets $G_i$ of filter coefficients to be determined as variables) would then be a direction that minimizes the L1-norm (maximizing sparsity) but also maximizes skewness. For example, the weights $cf_k$ may be determined based on the indicator BI so that the cost function CF is selected/composed from/of a set of K predefined cost functions $CF_k$. Selecting a certain cost function $CF_k$ can be carried out by setting the respective weight to any value $cf_k \neq 0$. To deselect a certain cost function CFk, the processor 33 may set the respective weight to the value $cf_k = 0$.

The method 39 may comprise a step 51 for defining one or more constraints to be used when optimizing the sets $G_i$ of filter coefficients. Such constraints, e.g. in the form of equalities or inequalities, place limits on the solution space, i.e. the mathematical domain in which the optimization algorithm used in a later step of the method 39 can search for an optimum value of the cost function CF.

For example, an equality constraint requiring the sum of the coefficients $G_i$ to be constant may be used to force the DC gain (gain at 0 Hz) of a filter 29 to be constant. A constraint of this type can be used when it is known that the gain at low frequency will not change when the measurement device 11 is in operation and forces the optimization algorithm to change the frequency response at higher frequencies only. Similarly, another constraint could be applied that forces the gain of a filter 29 at frequency other than DC to a constant value.

Furthermore, constraints can be defined to prevent one or more digital filters 29 of different channels 23 from becoming too similar to each other and attenuating the same frequency band of input signals $e_i$ while attempting to find the optimum point of the cost function CF. This could happen in a situation where the common mode signal is dominated by a single frequency (e.g. powerline frequencies of 50 Hz or 60 Hz). The optimization algorithm could place a zero in the frequency response of the individual digital filters 29 and thus remove the common-mode interference even before the vector signal $x_j$ is calculated, e.g. by subtracting two digital signals $d_i$ from each other. Removing the common-mode part by band-stop filtering rather than subtracting is not the goal of electrode equalization and would have a significant adverse effect on the difference signal of interest. This constraint may include a similarity measure to be calculated for the sets of filter coefficients of the respective digital filters. Instead of, or in addition to the similarity measure, gain constraints in inequality form may be used for frequencies inside the range of the signal of interest to avoid band-stop behavior.

Additionally or alternatively to using constraints to prevent the method 39 from choosing similar filter coefficients for different filters 29, the similarity measure related to the similarity of the filters 29 may also be included into the cost function CF.

Constraints may also be defined in order to limit some norm of the pulses of one or more filter 29 to a given value. Limiting this norm has the effect that the numerical stability of the filters 29 is improved by avoiding numerical overflow or saturation.

Based on the selected or composed target function CF and the constraints defined in step 51, step 53 of the method 39 performs an optimization operation to calculate new sets $G_{i,opt}$ of filter coefficients from the vector signal $x_j$ the sample (on-line mode) or sequence of samples (batch mode) of the unfiltered digital signal $d_i$ and the vector signal $x_j$. The new filter coefficients $G_{i,opt}$ constitute variables of an optimization problem to be solved in step 53. Because the delay caused by the digital filters 29 can be quite easily controlled (compared to analog filters) by appropriately choosing the set of coefficients $G_i$, the optimization step 51 will find a solution that reduces any adverse effect of the above-described time skew between digital signals $d_i$, on the effectiveness of common-mode interference mitigation.

When operating in on-line mode, known minimization algorithms such as stochastic gradient descent, in particular least mean squares (LMS) or recursive least squares (RLS) may be applied. In batch mode, known minimization algorithms such as deepest descent, Newton iteration or inner-point methods may be applied. Step 53, however, is not limited to a certain type of optimization algorithm. Although the optimization problem is formulated here as a cost minimization problem, an equivalent utility maximization problem may be formulated in steps 49 and 51 and solved in step 53.

After completion of the optimization run in step 53, the obtained new filter coefficients $G_{i,opt}$ may be applied to the individual digital filters 29 in step 55.

After step 55, the method 39 goes back to step 43 so that steps 43 to 55 are carried out iteratively either for each sample (on-line mode) or for the sequence of subsequent samples (batch mode) of the digital signals $d_i$.

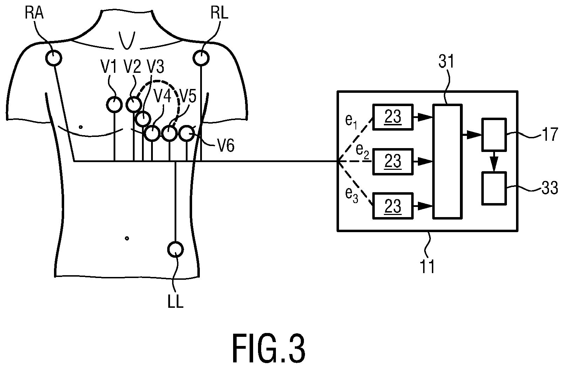

Although the herein described principle of the measurement device 11 and method 39 can be applied to any type of electrophysiological measurement procedures like EEG or EMG, in the following a more detailed example related to ECG measurements is given. This example includes a free channel ECG measurement device 11, the channels corresponding to three limb electrodes (right arm RA, left arm LA, left leg LL). As illustrated in Fig. 3, the three electrodes LA, LA, LL correspond to the analog electrical signals e1, e2, e3, respectively. This example can be extended as will be described below by adding further channels and electrodes such as six chest electrodes (V1, ..., V6).

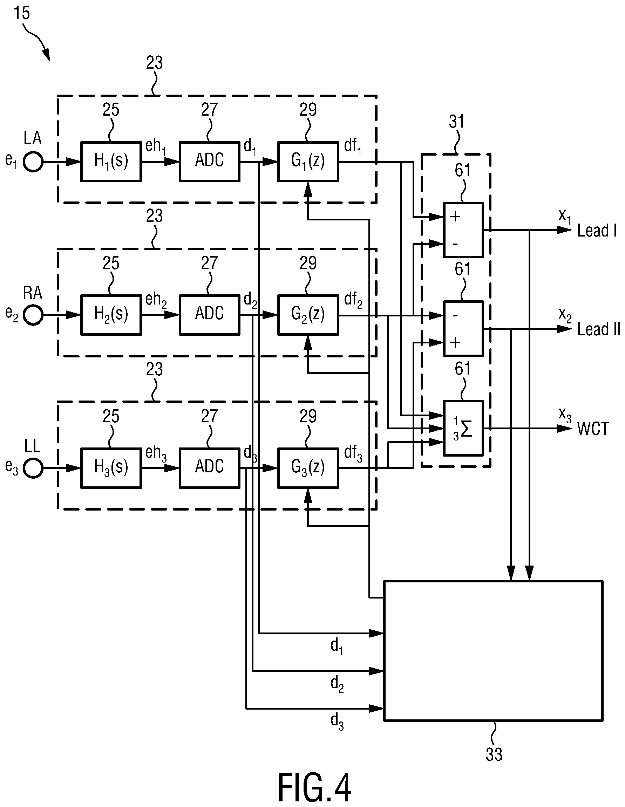

Fig. 4 shows a block diagram of the signal acquisition front end 15 of the three channel ECG device illustrated in Fig. 3. The three channel front end 15 has the same structure as the front end 15 shown in Fig. 1 but a further channel 23 has been added. Due to the higher number of channels, more than one vector signal $x_i$ may be defined. In the here described ECG application, three vector signals $x_1$, $x_2$, $x_3$ correspond to three leads referred to as "Lead I", "Lead II" and "WCT", respectively. WCT stands for the Wilson Central Terminal, which is a virtual potential calculated as the average of LA, RA and LL. In the shown example, the processor 33 performs optimization for the two leagues $x_1$, $x_2$ simultaneously, thereby optimizing the coefficients $G_i$ of filters 29 of all channels 23 that are used to calculate the respective vector signals $x_1$, $x_2$. Although at least one channel 23, such as the channel 23 related to the input signal $e_2$ and the electrode RA, is used to obtain multiple vector signals, there is only one channel filter 29 assigned to that channel. In other words, different vector signals $x_j$ that are determined based on the digital signal $df_j$ of the same channel 23 share the digital filter 23 of that channel.

As can be seen in Fig. 4, the combiner 35 as three separate combining elements 61 assigned to the individual vector signals $x_j$. In particular, $x_1$ (Lead I) is calculated by subtracting the digital signal $df_2$ related to the electrode RA of the digital signal $df_1$ related to the electrode LA. The vector signal $x_2$ is calculated by subtracting the digital signal $df_2$ related to the electrode RA from the digital signal $df_3$ related to the electrode LL.

Furthermore, it should be noted that it is not necessary to apply the above-described optimization method 39 to each vector signal $x_j$. In the shown example, the vector signal $x_3$ (WCT) is excluded from optimization, in particular the cost function CF is not calculated based on this vector signal $x_3$.

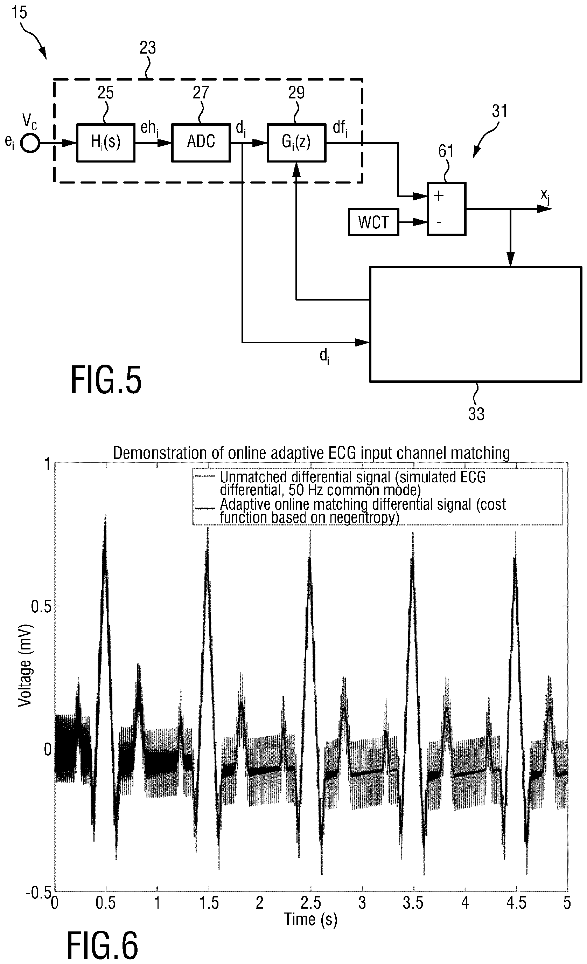

In a further example depicted on Fig. 5, the measurement device 11 shown in Fig. 3 and Fig. 4 is extended so that it can calculate further vector signals $x_j$, j = 4, ..., M based on electrical signals $e_i$, i = 4, ..., M inputted from the chest electrodes V1-V6. Accordingly, the front end 15 comprises at least one additional channel 23 assigned to one chest electrode. In case of a nine channel ECG device, six additional channels 23 are provided for the individual chest electrodes V1 to V6. Respective combining elements 61 of the signal combiner 31 calculate the difference between the filtered digital signal $df_i$ of the respective channel 23 and the virtual potential WCT corresponding to the vector signal $x_3$. For the sake of simplicity only one of these additional channels 23 and one respective combining element 61 is shown in Fig. 5. The respective vector signal $x_j$ is a further variable of the cost function CF and the respective unfiltered digital signal $d_i$ is used for calculating an optimized version of the respective filter coefficients $G_i$. No filtering is applied to the WCT-related vector signal $x_3$. The processor 33 performs the optimization method 39 described above and shown in Fig. 2. However, compared to the example of Fig. 1, the complexity of the optimization problem is increased due to the higher number of sets of filter coefficients $G_i$ and the increased number of vector signals $x_j$.

As can be seen in Fig. 4 and 5, the signal combiner 31 may include multiple combining elements 61. To calculate at least one vector signal $x_j$ from at least two processed digital signals $df_i$, a single combining element 61 may be used (e.g. for calculating the vector signals x1, x2, x3 corresponding to Lead I, Lead II and WCT) or multiple combining elements 61 may be combined, in particular cascaded (e.g. the vector signals xj shown in Fig. 5 related to the chest leads are calculated by the combining element 61 of Fig. 6 and the WCT combining element 61 shown in the bottom of Fig. 4). Combining and/or cascading combining elements 61 is particularly useful when different vector signals need to be calculated by the signal combiner 31 from a common vector signal such as the vector signal $x_3$ corresponding to the WCT.

Fig. 6 shows how the method 39 improves the signal quality when performing on-line optimization in the course of a measurement process. More specifically, the diagram of Fig. 6 shows the voltage in millivolt of a vector signal over time (in seconds). The vector signal obtained without applying the adaptive filters 29 is shown as a grey curve. The vector filter obtained when applying the filters 29 in the individual channels 23 is shown as a black curve in the diagram. As can be seen, the latter signal is superimposed by disturbing noise due to common mode interference at the beginning of the measurement (time t = 0). In the curse of the measurement the proportion of the disturbing noise gradually decreases while the method 39 continuously adapts the sets of filter coefficients $G_i$ of the digital filters 29.

To sum up, the herein described method 39 and the respective devices 11, 15 allow to iteratively adapt digital filters 29 present in the individual input channels 23 in order to reduce disturbances such as common mode interference. The filter coefficients $G_i$ are adapted based on a cost function related on statistical properties of at least one vector signal $x_j$. The electrode-specific adaptive filters 29 are iteratively tuned based on an optimization algorithm using a cost function composed of statistical properties of at least one vector signal, for example L1-norm, total variation, kurtosis, skewness or negentropy. The inventor has realized that good common-mode rejection can be obtained by minimizing the statistical similarity of the vector signal with usual common-mode signal patterns and/or maximizing the statistical similarity of the at least one vector signal with expected patterns of a desired signal such as expected ECG signal patterns.

**Claims**

1. Physiological measurement device (11) comprising a plurality of input channels (23), at least one of the input channels including a digital section configured to process at least one digital signal ($d_1$, $d_2$) representing an analog signal ($e_1$, $e_2$) present at an electrode (13) assigned to the respective channel, a signal combiner (31) configured to calculate at least one vector signal (x) from at least two processed digital signals ($df_1$, $df_2$), wherein the digital section of at least one input channel (23) comprises a digital filter (29) configured to filter the digital signal ($d_1$, $d_2$) based on a set of filter coefficients ($G_1$, $G_2$) and a processor (33) configured for calculating the set of filter coefficients ($G_1$, $G_2$) based on the vector signal ($x_1$).

2. Device (11) according to claim 1, wherein calculating the set of filter coefficients comprises calculating (49) at least one measure, preferably a statistical measure, of a block of subsequent time domain samples of the vector signal (x).

3. Device (11) according to claim 1 or 2, wherein calculating the set of filter coefficients ($G_1$, $G_2$) is further based on the unfiltered digital signal ($d_1$, $d_2$).

4. Device (11) according to one of the preceding claims, wherein calculating the set of filter coefficients is further based on a current set of filter coefficients (G1, G2) and wherein calculating the set of filter coefficients ($G_1$, $G_2$) is performed repeatedly for each newly available sample of the digital signal ($d_1$, $d_2$) or for a block of multiple subsequent samples of the digital signal ($d_1$, $d_2$).

5. Device (11) according to one of the preceding claims, wherein calculating the set of filter coefficients ($G_1$, $G_2$) comprises performing (53) an optimization step, the measure constituting a target function (CF) and the set of filter coefficients ($G_1$, $G_2$) constituting input variables of the target function.

6. Device (11) according to claim 5, wherein the target function (CF) comprises a weighted sum of multiple measures ($CF_K$).

7. Device (11) according to claim 5 or 6, wherein performing (53) the optimization step is subject to at least one constraint related to a certain set of filter coefficients ($G_1$, $G_2$).

8. Device (11) according to claim 7, wherein the constraint includes at least one equality constraint and/or at least one inequality constraint.

9. Device (11) according to one of claims 5 to 8, wherein performing (53) the optimization step is subject to at least one constraint related to a similarity measure characterizing a similarity of two different sets of filter coefficients ($G_1$, $G_2$).

10. Device (11) according to one of claims 5 to 9, wherein the target function (CF) comprises the similarity measure characterizing a similarity of two different sets of filter coefficients.

11. Device (11) according to one of the preceding claims, wherein the processor (33) is configured to calculate multiple vector signals ($x_1$, $x_2$, $x_3$, $x_j$) from the filtered digital signals ($df_1$, $df_2$, $df_3$, $df_i$), wherein the digital section of one channel (23) includes at most one digital filter (31).

12. Device (11) according to one of the preceding claims, wherein the processor (32) is configured to receive (49) an indication (BI) from a back end (17) and to determine the at least one measure and/or the target function based on the received indication.

13. Device (11) according to one of the preceding claims, wherein the signal combiner (31) is configured to calculate the vector signal ($x_j$) from a further vector signal ($x_3$).

14. Physiological measurement method (39) comprising processing at least one digital signal ($d_1$, $d_2$) representing an analog signal ($e_1$, $e_2$) present at an electrode (13) assigned to a digital section of one of a plurality of input channels (23) of a physiological measurement device (11);
    calculating at least one vector signal (x) from at least two processed digital signals ($df_1$, $df_2$),
    wherein processing the digital signal ($d_1$, $d_1$) comprises digitally filtering the digital signal ($di$, $d_2$) of at least one input channel (23) based on a set of filter coefficients ($G_1$, $G_2$) and
    wherein the method (39) comprises calculating the set of filter coefficients ($Gi$, $G_2$) based on the vector signal (x).

15. Computer program comprising program code means (37) for causing a computer (35) to carry out the steps of the method (39) as claimed in claim 14 when said computer program is carried out on the computer (35).

FIG.1

FIG.2

FIG.3

FIG.4

FIG.5

Demonstration of online adaptive ECG input channel matching

Legend:
- Unmatched differential signal (simulated ECG differential, 50 Hz common mode)
- Adaptive online matching differential signal (cost function based on negentropy)

FIG.6

Europäisches
Patentamt

European
Patent Office

Office européen
des brevets

# EUROPEAN SEARCH REPORT

Application Number

EP 21 16 2472

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 3 372 148 A1 (KONINKLIJKE PHILIPS NV [NL]) 12 September 2018 (2018-09-12) * paragraphs [0026], [0027], [0030] * ----- | 1-15 | INV. A61B5/305 A61B5/318 A61B5/00 |
| X | EP 2 394 571 A1 (SAMSUNG ELECTRONICS CO LTD [KR]) 14 December 2011 (2011-12-14) * paragraphs [0056], [0057], [0065] * ----- | 1-15 | |

TECHNICAL FIELDS
SEARCHED      (IPC)

A61B

The present search report has been drawn up for all claims

| Place of search | Date of completion of the search | Examiner |
|---|---|---|
| The Hague | 31 August 2021 | Knüpling, Moritz |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

        .................................................................................

& : member of the same patent family, corresponding
    document

EPO FORM 1503 03.82 (P04C01)

## ANNEX TO THE EUROPEAN SEARCH REPORT
## ON EUROPEAN PATENT APPLICATION NO.

EP 21 16 2472

This annex lists the patent family members relating to the patent documents cited in the above-mentioned European search report.
The members are as contained in the European Patent Office EDP file on
The European Patent Office is in no way liable for these particulars which are merely given for the purpose of information.

31-08-2021

| Patent document cited in search report | | Publication date | Patent family member(s) | | Publication date |
|---|---|---|---|---|---|
| EP 3372148 | A1 | 12-09-2018 | EP 3372148 A1 | | 12-09-2018 |
| | | | EP 3592207 A1 | | 15-01-2020 |
| | | | US 2020000411 A1 | | 02-01-2020 |
| | | | WO 2018162365 A1 | | 13-09-2018 |
| EP 2394571 | A1 | 14-12-2011 | CN 102283641 A | | 21-12-2011 |
| | | | EP 2394571 A1 | | 14-12-2011 |
| | | | JP 5898863 B2 | | 06-04-2016 |
| | | | JP 2011255187 A | | 22-12-2011 |
| | | | KR 20110135296 A | | 16-12-2011 |
| | | | US 2011306892 A1 | | 15-12-2011 |

EPO FORM P0459

For more details about this annex : see Official Journal of the European Patent Office, No. 12/82

**EP 4 059 430 A1**

**Patent documents cited in the description**

- DE 102014214994 A1